# EUROPÄISCHE PATENTANMELDUNG

(11) **EP 1 013 220 A1**
(43) Veröffentlichungstag der Anmeldung: **28.06.2000**
(21) Anmeldenummer: 99125489.7
(22) Anmeldetag: 21.12.1999
(51) Int. Cl.: A61B 5/022

(54) **Spannvorrichtung für die Manschette eines Blutdruckmessgerätes**

(30) Priorität: 22.12.1998 DE 19859392
(71) Anmelder: Braun GmbH, 61476 Kronberg (DE)
(72) Erfinder: Freund, Dirk, 65779 Kelkheim (DE); Hollinger, Stefan, 61476 Kronberg (DE); Rönneberg, Gerrit, 64289 Darmstadt (DE); Giersiepen, Martin, 61440 Oberursel (DE); Kressmann, Frank, 65824 Schwalbach (DE); Schnak, Fred, 61476 Kronberg (DE)

(57) **Zusammenfassung**

Es wird ein Verfahren zum Anlegen einer Manschette eines Blutdruckmeßgerätes um ein Körperglied, insbesondere ein Handgelenk, beschrieben, bei dem die Manschette (2) derart unter Aufbau einer Spannkraft um das Körperglied gelegt wird, dass durch Expansion einer in die Manschette integrierten flexiblen Druckblase (66) ein blutdurchströmtes Körpergefäß abdruckbar ist. Nach der Erfindung wird die mittels eines motorischen oder manuell betätigbaren Spannantriebes (10,45,68,92) aufgebrachte Spannkraft automatisch auf einer vorgebbare Sollspannkraft begrenzt. Dadurch läßt sich unabhängig vom Anbringungsort der Manschette und/oder von der vom Bediener oder einem Motor des Spanntriebs aufgebrachten Kraft jederzeit reproduzierbar eine zur Blutdruckmessung besonders geeignete Manschettenspannung einstellen. Mit erfindungsgemäß ausgebildeten Blutdruckmeßgeräten können bedienerunabhängig gut reproduzierbare und damit diagnostisch gut verwendbare Blutdruckmeßergebnisse erzielt werden.

## Beschreibung

Die Erfindung betrifft ein Verfahren zum Anlegen einer Manschette eines Blutdruckmeßgerätes um ein Körperglied, insbesondere ein Handgelenk, bei dem die Manschette derart unter Aufbau einer Spannkraft um das Körperglied gelegt wird, daß durch Expansion einer der Manschette zugeordneten, insbesondere in die Manschette integrierten, fluidbefüllbaren Druckblase ein blutdurchströmtes Körpergefäß abdrückbar ist.

Die Erfindung betrifft weiterhin eine Spannvorrichtung für eine um ein Körperglied, insbesondere ein Handgelenk, legbare Manschette eines Blutdruckmeßgerätes gemäß dem Oberbegriff des Anspruchs 13. Die Erfindung betrifft außerdem ein Blutdruckmeßgerät gemäß dem Oberbegriff des Anspruchs 27.

Die meisten gebräuchlichen Blutdruckmeßgeräte weisen eine flexible oder mit starren, ggf. gelenkig miteinander verbundenen Teilen aufgebaute Manschette auf, die unter Aufbau einer Spannkraft um ein Körperglied, beispielsweise einen Oberarm oder ein Handgelenk, gelegt wird. Zur Messung des arteriellen Blutdrucks wird eine der Manschette zugeordnete, insbesondere in die Manschette integrierte, flexible Druckkammer bzw. Druckblase derart aufgepumpt, daß durch ihre Expansion eine Arterie bis zur vollständigen Undurchlässigkeit abgedrückt wird. Anschließend wird das Fluid aus der Druckblase langsam abgelassen und der Fluiddruck kontinuierlich wieder auf Atmosphärendruck reduziert. Dabei tritt durch den Puls eine Oszillation des Fluiddrucks in der Druckblase auf, aus der sich bei der oszillometrischen Messung z.B. der systolische und der diastolische Blutdruck bestimmen lassen.

Das Anlegen und Spannen der Manschette erfolgt von Hand, beispielsweise mittels eines durch eine Schlaufe gezogenen Spanngurtes, dessen freies Ende nach Festzurren mittels textiler Haftverbinder festgelegt wird oder mittels Schnallenverschluß mit Längeneinstellung. Es sind auch schon Spannvorrichtungen mit integriertem Spannschieber bekannt geworden. Aus dem deutschen Gebrauchsmuster DE 297 19 501 ist eine Manschette mit einer manuell betätigbaren Spannvorrichtung bekannt, die es dank eines verstellbaren Anschlagselementes oder einer individuell verstellbaren Markierung einem Benutzer ermöglicht, eine einmal für ausreichend empfundene Manschettenweite immer wieder ohne Aufwand zu reproduzieren.

Es hat sich bei Untersuchungen gezeigt, daß mit diesen herkömmlichen Blutdruckmeßgeräten bei mehrfachem Anlegen der Manschette an unterschiedlich dicken Handgelenken und wiederholten Messungen Meßwertschwankungen vorkommen können, die häufig auf Blutdruckwerten basierende Aussagen über den Zustand eines Patienten erschweren oder gar unmöglich machen.

Demgemäß liegt der Erfindung die Aufgabe zugrunde, Maßnahmen und Mittel vorzuschlagen, mit denen sich die Meßgenauigkeit der beschriebenen Blutdruckmessung erhöhen läßt und insbesondere reproduzierbare Meßergebnisse erhältlich sind.

Zur Lösung dieser Aufgabe schlägt die Erfindung ein Verfahren mit den Merkmalen von Anspruch 1 sowie eine Spannvorrichtung mit den Merkmalen von Anspruch 13 und ein Blutdruckmeßgerät mit den Merkmalen des Anspruchs 27 vor.

Bei einem Verfahren der eingangs genannten Art wird diese Aufgabe erfindungsgemäß dadurch gelöst, daß die Spannkraft automatisch auf eine vorgebbare Sollspannkraft begrenzt wird. Die automatische, also nicht vom Verhalten des Bedieners beim Anlegen und Spannen abhängige Spannkraftbegrenzung sorgt dafür, daß die Manschette stets mit einer reproduzierbaren Spannkraft um das Körperglied gelegt wird, gleichgültig wie das Körperglied beschaffen ist. So wird also beispielsweise bei Handgelenk-Meßgeräten ermöglicht, daß die Manschettenspannkraft unabhängig davon ist, ob die Manschette an einer etwas dünneren Stelle oder an einer etwas dickeren Stelle des Handgelenkbereiches angelegt wird. Denn es wird insbesondere nicht die Manschettenweite vorgegeben, sondern eine bestimmte Sollspannkraft, die sich unabhängig von der Manschettenweite beim Festspannen automatisch ergibt. Das Problem, daß manche Bediener eine Manschette eher zu locker, andere sie dagegen eher zu fest gespannt anlegen, wird durch die Erfindung beseitigt. Es hat sich gezeigt, daß bedienerunabhängige sehr gut reproduzierbare Meßergebnisse erzielt werden können. Eine derartige Spannkraftbegrenzung ist insbesondere vorteilhaft für Geräte, die außerhalb ärztlicher Praxen von in der Regel unerfahrenen Anwendern zur Selbstkontrolle verwendet werden.

Die Spannkraft kann manuell erzeugt werden, beispielsweise durch unmittelbares Ziehen am Ende der Manschette oder mittels einer handbetätigbaren Spannvorrichtung, die beispielsweise ein mit der Manschette gekoppeltes Betätigungsglied wie ein griffgünstig gestaltetes Stellrad, einen Kipp- oder Drehhebel oder einen Schieber o. dgl. aufweisen kann. Derartige Lösungen können kostengünstig und robust realisiert werden.

Es ist auch möglich, daß die Spannkraft automatisch, insbesondere elektromotorisch und/oder pneumatisch und/oder hydraulisch, erzeugt wird. Ausführungen mit motorisch betriebenen Spannvorrichtungen sind bequem zu handhaben und erfordern beim Bediener wenig Kraftaufwand, was insbesondere bei älteren Patienten, die unter Umständen mehrmals täglich selbständig eine Blutdruckmessung durchführen müssen, von Vorteil ist.

Eine automatische, insbesondere motorische Unterstützung oder Durchführung des Anlegens und/oder Spannens der Manschette und der Manschettenweitenverringerung bzw. Manschettenspannung kann auch unabhängig von der automatischen Spannkraftbegrenzung vorteilhaft sein und beispielsweise bis zum Schließen auf eine vorgebbare Manschettenweite genutzt werden.

Es gibt mehrere Möglichkeiten, eine automatische Spannkraftbegrenzung zu realisieren. Es kann beispielsweise so sein, daß zum Schließen bzw. Verengen der Manschette und zur Erzeugung der Manschettenspannung mindestens ein mit der Manschette kraftübertragend gekoppelter, manueller oder automatischer Spannantrieb betätigt wird und daß der Spannantrieb bei Erreichen der Sollspannkraft von der Manschette kraftentkoppelt wird. Es kann also eine kraft - oder momentbegrenzte Kraftübertragung zwischen Spannantrieb und Manschette vorgesehen sein, die die Kraft nur bis zu einer der Sollspannkraft entsprechenden Sollkraft überträgt, darüber hinausgehende Kräfte jedoch nicht weiterleitet. Insbesondere kann im Kraftfluß eine Rutschkupplung oder eine Drehmomentbegrenzung o. dgl. eingebaut sein. Es ist alternativ oder zusätzlich auch möglich, eine bei Erreichen der Sollspannkraft automatisch eingreifende Verriegelung vorzusehen, die eine weitere Kraftübertragung zwischen Spannantrieb und Manschette verhindert.

Eine alternativ oder zusätzlich realisierbare Weiterbildung sieht vor, daß die Spannkraft mit einem automatischen bzw. motorisch betriebenen Spannantrieb erzeugt wird und daß bei Erreichen der Sollspannkraft der Spannantrieb angehalten, insbesondere abgeschaltet wird. Derartige Ausführungsformen können ohne kraftabhängig schaltende Entkopplungen auskommen.

Ganz besonders vorteilhaft kann es sein, wenn die einen automatischen Spannantrieb antreibende Kraft nach Erreichen der Sollspannkraft teilweise oder vollständig auf eine oder mehrere, von der Manschette gesonderte andere Komponenten des Blutdruckmeßgerätes umgeleitet wird, wodurch eine Mehrfachnutzung des motorischen Antriebes der Spannvorrichtung möglich wird bzw. eine ohnehin vorhandene automatische Kraftquelle sowohl für die Manschettenspannung, als auch für andere Zwecke nutzbar wird. Beispielsweise kann eine zum Befüllen der Druckblase ohnehin vorgesehene Druckerzeugungseinrichtung zunächst einen pneumatischen Spannantrieb bis zum Erreichen der Sollspannkraft antreiben und danach, nach geeigneter Umleitung des Druckfluides, die expandierbare Druckblase befüllen.

Bei bestimmten Weiterbildungen kann vorgesehen sein, daß beim Spannen der Manschette mindestens eine der auf die Manschette wirkenden Spannkraft entsprechende Kenngröße gemessen bzw. erfaßt wird und daß bei Erreichen einer der Sollspannkraft entsprechenden Sollkenngröße das Spannen bzw. das Schließen oder Verengen der Manschette abgebrochen bzw. beendet wird. Die mittels eines oder mehrerer geeigneter Sensoren zu erfassende Kenngröße kann beispielsweise der Druck in einem Pneumatikzylinder eines pneumatischen Spannantriebes und/oder der Leistungsverbrauch bzw. Motorstrom eines den Spannantrieb antreibenden Elektromotors sein oder der mit abnehmender Manschettenweite ab einem gewissen Manschettenumfang relativ rasch zunehmende Druck in einer teilweise fluidbefüllten Druckblase. Es können auch mechanische Kenngrößen, beispielsweise die Durchbiegungen oder Auslenkung eines federelastischen Elementes o. dgl. erfaßt und zur Spannkraftbegrenzung genutzt werden.

Das mit einer Verringerung der Manschettenweite einhergehende Schließen oder Spannen der Manschette kann auf jede geeignete Art durchgeführt werden. Beispielsweise können gelenkig miteinander verbundene, in sich starre Manschettenabschnitte gegeneinander verschwenkt werden oder es kann ein flexibler oder starrer Manschettenabschnitt durch eine Spannvorrichtung hindurchgezogen oder in eine Spannvorrichtung eingezogen werden. Bei einer bevorzugten Ausführungsform wird zum Schließen bzw. Spannen der Manschette mindestens ein flexibler Manschettenabschnitt, insbesondere mindestens ein Manschettenendabschnitt, aufgewickelt. Hierdurch läßt sich eine besonders platzsparende Unterbringung überschüssiger Manschettenlängen erreichen. Die Möglichkeit einer Aufwicklung der Manschette kann insbesondere auch dazu genutzt werden, daß die Manschette zur Verwahrung im wesentlichen vollständig, insbesondere motorisch aufgewickelt wird, was einerseits Beschädigungen der Manschette bei Transport oder Verwahrung vermeiden hilft und eine platzsparende Verwahrung einer nicht benötigten Manschette ermöglicht.

Die Genauigkeit und Reproduzierbarkeit der Blutdruckmessung läßt sich bei Weiterbildungen der Erfindung besonders dadurch verbessern, daß bei oder nach Erreichen der Sollspannkraft die zugeordnete Manschettenweite festgelegt bzw. festgesetzt wird, wodurch eine unbeabsichtigte, die Meßergebnisse möglicherweise verfälschende Aufweitung der Manschette während der Messung zuverlässig verhindert wird. Die Festlegung der Manschettenweite kann formschlüssig erfolgen, beispielsweise mittels einer Sicherung in Form eines ggf. manuell wieder entriegelbaren Sperrriegels oder Sperrhebels. Bei einer besonders bevorzugten Ausführungsform erfolgt die Festlegung reibschlüssig, insbesondere ohne daß gesonderte Verriegelungselemente vorgesehen sein müssen. Hierzu kann die Kraftübertragung zwischen Spannantrieb und Manschette einen Gewindetrieb mit Selbsthemmung aufweisen, der beispielsweise in Form eines Schneckenzahnstangengetriebes ausgeführt sein kann.

Zum Öffnen bzw. Erweitern der Manschette z.B. nach erfolgter Messung kann eine ggf. vorhandene Verriegelung gelöst und die Manschette manuell aufgezogen bzw. erweitert werden. Besonders bequem sind gattungsgemäße oder erfindungsgemäße Ausführungen, bei denen die Öffnung bzw. Erweiterung der Manschette motorisch durchgeführt oder unterstützt wird, vorzugsweise bis zu einer maximalen Öffnungsweite der Manschette. Als Öffnungsantrieb kann beispielsweise ein Motor eines Spannantriebs genutzt werden, der zum Öffnen insbesondere in umgekehrter Drehrichtung angetrieben werden kann. Bei einem Gleichstrommotor kann dies vorteilhaft durch Umpolung der Versorgungsspannung erfolgen.

Eine besondere Weiterbildung erweitert die Nutzungsmöglichkeiten erfindungsgemäßer Blutdruckmeßgeräte erheblich. Bei dieser ist vorgesehen, daß bei Erreichen der Sollspannkraft ein der zugeordneten Manschettenweite zugeordneter Meßwert bestimmt wird und aus dem Meßwert mindestens ein Dimensionsparameter des von der Manschette umschlungenen Körpergliedes abgeleitet wird, insbesondere ein Körperglieddurchmesser und/oder ein Körpergliedumfang. Die Spannkraftbegrenzung ermöglicht hierbei auch bei naturgemäß komprimierbaren Körpergliedern gut reproduzierbare Meßergebnisse, da auf das Körperglied immer im wesentlichen die gleiche Kompressionskraft wirkt. Der mindestens eine Meßwert kann beispielsweise zur Korrektur von Blutdruckmeßwerten und/oder zur Identifizierung des Benutzers herangezogen bzw. verwendet werden.

Eine zur Durchführung des Verfahrens geeignete Spannvorrichtung für eine um ein Körperglied, insbesondere um ein Handgelenk legbare Manschette eines Blutdruckmeßgerätes ist derart ausgebildet, daß die Manschette unter Aufbau einer Spannkraft derart um das Körperglied legbar ist, daß durch Expansion einer der Manschette zugeordneten, insbesondere in eine, vorzugsweise flexible, Manschette integrierten fluidbefüllbaren Druckblase ein blutdurchströmtes Körpergefäß abdrückbar ist. Eine erfindungsgemäße Spannvorrichtung ist gekennzeichnet durch Begrenzungsmittel zur automatischen Begrenzung der Spannkraft auf eine vorgebbare Sollspannkraft.

Im Zusammenhang mit den Ausführungsformen wird näher erläutert, daß die Begrenzungsmittel mindestens eine in eine kraftübertragende Kopplung zwischen einem Spannantrieb und der Manschette eingefügte Kraftübertragungsbegrenzungseinrichtung aufweisen können, die insbesondere als kraftabhängig eingreifende Entkopplungseinrichtung zwischen Spannantrieb und Manschette ausgebildet und/oder bezüglich der Kraft, bei der sie eingreift, einstellbar sein kann. Entkopplungseinrichtungen wie Drehmomentbegrenzungen, mechanische Rutschkupplungen o. dgl. sind insbesondere mit manuell betätigbaren Spannantrieben vorteilhaft, da ein Bediener normalerweise die richtige Spannkraft manuell nur unzureichend genau einstellen bzw. reproduzieren kann. Eine als Rutschkupplung ausgebildete Entkopplungseinrichtung wird im Zusammenhang mit einer Ausführungsform näher beschrieben.

Eine Weiterbildung, die insbesondere im Zusammenhang mit motorischen Spannantrieben vorteilhaft ist, zeichnet sich dadurch aus, daß die Begrenzungsmittel eine Einrichtung zum spannkraftabhängigen Anhalten, insbesondere Abschalten des Spannantriebes aufweisen. Eine im Zusammenhang mit einer pneumatisch arbeitenden Spannvorrichtung näher erläuterte, besondere Weiterbildung sieht vor, daß Mittel zum Umlenken der einen Spannantrieb antreibenden Kraft von der Manschette auf mindestens eine andere Einrichtung des Blutdruckmeßgerätes bei Erreichen der Sollspannkraft vorgesehen sind, wobei die Mittel zum Umlenken vorzugsweise bei Erreichen der Spannkraft automatisch ohne Zutun eines Benutzers eingreifen und eine vorteilhafte Doppelnutzung eines beispielsweise elektromotorisch und/oder pneumatisch arbeitenden Antriebes ermöglichen.

Eine alternativ oder zusätzlich einsetzbare Möglichkeit sieht mindestens eine Sensoreinrichtung zur Erfassung mindestens einer der an der Manschette angreifenden Spannkraft zugeordneten Kenngröße vor sowie mindestens eine Einrichtung zur Beendigung des Spannens bei Erreichen einer der Sollspannkraft entsprechenden Sollkenngröße. Die Sensoreinrichtung kann beispielsweise einen Dehnungsmeßstreifen, einen elektrokapazitiv oder resistiv arbeitenden Sensor oder mindestens einen Drucksensor aufweisen, der den Druck in einem Hydraulikzylinder oder Pneumatikzylinder eines Stellantriebes überwacht. Vorteilhaft kann hierzu ein ohnehin vorhandener Drucksensor genutzt werden, der zur Überwachung des Druckblasendruckes bei der Blutdruckmessung vorgesehen sein muß. Alternativ oder zusätzlich kann als Kenngröße z.B. auch die Leistungsaufnahme bzw. der Motorstrom eines Elektromotors des Spannantriebes genutzt werden.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein können. Ausführungsbeispiele der Erfindung sind in den Zeichnungen dargestellt und werden im folgenden näher erläutert. In den Zeichnungen zeigen:
- Fig. 1: eine perspektivische Darstellung einer Schließvorrichtung für eine Manschette eines Handgelenk-Blutdruckmeßgerätes mit einem Stellrad zur manuellen Betätigung einer Manschettenspannvorrichtung,
- Fig. 2: eine perspektivische Ansicht von Teilen der zugehörigen Manschettenspannvorrichtung,
- Fig. 3: eine weitere perspektivische Ansicht von Teilen der Manschettenspannvorrichtung,
- Fig. 4: eine Draufsicht auf einen zur Begrenzung der Spannkraft vorgesehenen Drehmomentbegrenzer der Ausführungsform nach Figuren 1 bis 3,
- Fig. 5: eine perspektivische Gesamtansicht einer Manschette mit einer Ausführungsform einer elektromotorisch betriebenen Spannvorrichtung,
- Fig. 6: eine vergrößerte Detailansicht der kraftübertragenden Kopplung zwischen Elektromotor und Manschette in Fig. 5 mit einem Zahnradsatz und einem Schneckentrieb mit Selbsthemmung,
- Fig. 7: eine schematische, perspektivische Ansicht einer anderen Ausführungsform einer Handgelenkmanschette mit einer pneumatischen Spannvorrichtung und
- Fig. 8: eine verschlußfreie Manschette mit einer elektromotorisch betriebenen Spannvorrichtung, die eine Aufwicklung der Manschette ermöglicht.

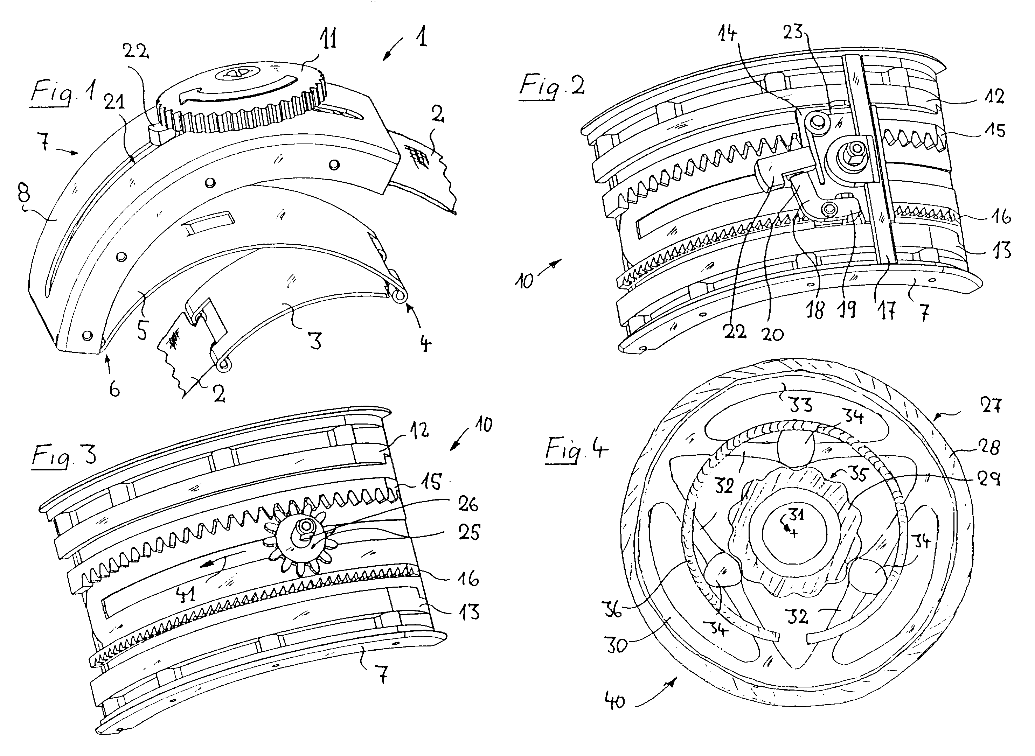

Die perspektivische Darstellung in Fig. 1 zeigt eine Schließvorrichtung 1 einer um ein Handgelenk zu legenden, flexiblen, bandförmigen Gewebeband-Manschette 2 eines Blutdruckmeßgerätes zur vorzugsweise oszillatorischen Messung des Blutdrucks. Die Schließvorrichtung hat ein bogenförmig gekrümmtes, bandförmiges erstes Gelenkteil 3, an das ein Endabschnitt der Manschette 2 angelenkt ist. Das Gelenkteil 3 ist über ein Schwenkgelenk 4 mit einem ebenfalls bandförmigen, bogenförmig gekrümmten, längeren Mittelgelenkteil 5 verbunden, das an der dem Gelenk 4 gegenüberliegenden Seite über ein weiteres einachsiges Schwenkgelenk 6 mit einem oberen Gelenkteil 7 verbunden ist, auf das unter Bildung eines flachen, gekrümmten Innenraumes eine Abdeckkappe 8 aufgeschraubt oder festgeschnappt ist. In dem Innenraum des oberen Gelenkteiles 7 befinden sich die in den Figuren 2 bis 4 gezeigten Elemente einer Manschettenspannvorrichtung 10, die über ein im montierten Zustand auf der Oberseite der Schließvorrichtung sitzendes, an seinem Umfang griffgünstig zahnradartig ausgestaltetes Stellrad 11 manuell betätigbar ist.

Die in den Figuren 2 bis 4 gut zu erkennende Spannvorrichtung 10 hat einen durch das obere Gelenkteil 7 gebildeten Träger mit zwei parallel zur Längsrichtung des Trägers verlaufenden, der Krümmung des Trägers folgenden Führungsschienen 12, 13 zur Führung eines in Längsrichtung des Trägers verschiebbaren Schlittens 14. Weiterhin sind an dem Träger eine parallel zu den Führungsschienen zwischen den Führungsschienen verlaufende erste Zahnstange 15 mit einer symmetrischen, groben Zahnung und eine parallel dazu verlaufende zweite Zahnstange 16 mit einer vergleichsweise feiner gerasteten unsymmetrischen sägezahnartigen Zahnung vorgesehen. An dem Schlitten 14 ist ein quer zur Längsrichtung des Trägers verlaufender, gerader Manschettenhalter 17 befestigt, an dem ein Ende der flexiblen Manschette 2 befestigbar ist. Auf dem Führungsschlitten 14 ist weiterhin eine L-förmig gewinkelte Sperrklinke 18 drehbar gelagert, deren Klinkenabschnitt 19 zum Eingreifen in die sägezahnförmige Zahnung der zweiten Zahnstange ausgebildet ist. Am gegenüberliegenden Hebelabschnitt 20 der Sperrklinke 18 ist ein im zusammengebauten Zustand (Fig. 1) unterhalb des Stellrades 11 durch einen Längsschlitz 21 der Kappe 8 hindurchragender Hebel 22 befestigt. Eine am Schlitten 14 befestigte Feder 23 drückt bei nicht gedrücktem Hebel 22 den Klinkenabschnitt 19 in die Kerbverzahnung der zweiten Zahnstange 16. Der formschlüssige Eingriff kann durch Drücken des Hebels 22 und Schwenken der Klinke 18 entgegen der Kraft der Feder 23 gelöst werden.

In dem Schlitten 14 ist ein in Fig. 3 gut zu erkennendes Zahnrad 25 drehbar gelagert, das im zusammengebauten Zustand zwischen Schlitten und Träger auf Höhe der ersten Zahnstange 15 angeordnet ist und mit dieser kämmt. Eine im Oberabschnitt seitlich abgeflachte Welle 26 des Zahnrades ragt im zusammengebauten Zustand der Vorrichtung durch den Längsschlitz 21 nach oben und trägt das Stellrad 11, das im zusammengebauten Zustand mit dem Zahnrad verbunden ist.

Um die Manschette immer mit einer ganz bestimmten, vorgegebenen Spannkraft anlegen zu können, ist das Stellrad mehrteilig ausgeführt, was anhand Fig. 4 näher erläutert wird. Ein Stellradoberteil 27 hat einen umlaufenden, ringförmigen Rand 28 und konzentrisch mit dem Rand in seinem Inneren ein mit einer gewellten Außenprofilierung versehenes, ansonsten rundes Innenteil 29, das einstückig mit dem Rand 28 ausgebildet ist. Durch den Rand 28 wird ein zwischen Rand und Innenteil angeordnetes Stellradunterteil 30 dicht umschlossen, das relativ zum Oberteil 27 um eine zentrische Achse 31 drehbar im Oberteil geführt ist. Oberteil 27 und Unterteil 30 bilden eine Baugruppe, die im zusammengebauten Zustand auf dem Zahnrad befestigt ist, wobei das Unterteil 30 drehfest mit dem Zahnrad mit einer am Außenring 33 angebrachten Flanschverbindung mit Verdrehschutz (Mitnehmerflächen 26 sind am Zahnrad 25 dargestellt) verbunden ist. Am Unterteil 30 sind drei um jeweils 120 umfangsversetzte, identische koplanare Federarme 32 einstückig ausgebildet. Diese verlaufen jeweils nach Art von Sehnen exzentrisch durch das Unterteil, sind jeweils an ihren beiden Enden mit dem ringförmigen Außenteil 33 des Unterteils 30 verbunden und tragen jeweils mittig im federelastischen Bereich Rastnoppen 34. Diese sind entsprechend den Federarmen jeweils um 120 umfangsversetzt zueinander angeordnet und greifen in der gezeigten Relativstellung von Oberteil und Unterteil in an der gewellten radialen Außenseite des Innenteils vorgesehene, gerundete Rastvertiefungen 35 ein, deren Umfangsabstand jeweils einen ganzzahligen Bruchteil vom Umfangsabstand der Rastnoppen entspricht, im Beispielsfall jeweils 30. Dadurch ist gewährleistet, daß alle Rastnoppen jeweils im gleichen Eingriffszustand mit dem gewellten Innenring 29 stehen. Bei der gezeigten Ausführungsform werden die Rastnoppen 34 noch durch einen um die radialen Außenseite der Rastnoppen herumgelegten, einseitig offenen Spannring 36 umschlossen, der eine zusätzliche in Eingriffsrichtung wirkende Federkraft bewirkt.

Durch diese Ausbildung ist in dem Stellrad 11 eine Kraftübertragungsbegrenzungseinrichtung bzw. Drehmomentbegrenzungseinrichtung 40 nach Art einer Rutschkupplung geschaffen, bei der das Unterteil 30 ein erstes Kupplungsglied und das Oberteil 27 ein zweites, gegen dieses verdrehbares Kupplungsglied bilden. Diese kraftabhängig auskoppelnde Entkopplungseinrichtung 40 kann wie folgt arbeiten. Zunächst wird die Manschette bei geöffneter Schließvorrichtung 1 und/oder teilweise oder voll aus der Spannvorrichtung ausgezogenen Manschette nach Durchführung einer Hand durch die Manschette um ein Handgelenk gelegt und die Schließvorrichtung geschlossen. Anschließend wird die Manschette manuell gespannt, indem das Stellrad 11 im Uhrzeigersinn (Pfeil) gedreht wird. Dabei wird das manuell aufgebrachte Drehmoment über die Rutschkupplung 40 auf das Zahnrad 26 übertragen, das sich entsprechend in Spannrichtung 41 (Fig. 3) entlang der Zahnradstange 14 unter Durchmesserverringerung der Manschette bewegt. Die Sperrklinke 18 läuft dabei von Seiten der Schrägflächen der Zähne der zweiten Zahnstange klickend über diese hinweg. Wenn die Manschette mit einer durch die Auslegung der Rutschkupplung 40 festlegbaren Sollspannkraft um den Arm gespannt ist, weichen die Federarme 32 unter Aufspreizung des Federringes 36 und radialer Auswärtsbewegung der Rastnoppen 34 aus und diese geraten außer Eingriff mit den Rastvertiefungen 35 am Innenring 29 des Oberteils 27, so daß Oberteil und Unterteil relativ gegeneinander verdreht werden können, ohne daß die Manschette weiter gespannt wird. Ein unfreiwilliges Entspannen der Manschette wird dadurch verhindert, daß die Feder 23 den Klinkenabschnitt 19 der Klinke 18 in die Kerbverzahnung der zweiten Zahnstange 16 drückt, so daß ein formschlüssiger Eingriff eine Rückbewegung verhindert und somit den Führungsschlitten 14 in der jeweiligen Position hält und die der Sollspannkraft entsprechende Manschettenweite festlegt. Soll die Manschette bewußt entsperrt werden, so muß der Hebel 22 gedrückt werden, wodurch die Sperrverzahnung freigegeben wird und der Führungsschlitten 14 entgegen Richtung 41 zurückgeschoben werden kann. Die durch die Rutschkupplung automatisierte Begrenzung der manuell aufgebrachten Spannkraft führt dazu, daß auch bei unterschiedlich großen Armdurchmessern immer ein optimaler Festsitz der Manschette gewährleistet ist, wodurch sich gut reproduzierbare Meßergebnisse ergeben.

Die Ausführungsform der Rutschkupplung 40 in Fig. 4 entkoppelt erst bei einem relativ großen Drehmoment. Um eine niedrigere Sollspannkraft einzustellen, kann beispielsweise eine Rutschkupplung ohne den Spannring 36 ausgeführt sein und/oder es ist auch möglich, statt der zweiseitigen Anbindung der Federarme nur eine einseitige Anbindung vorzusehen, so daß die Rastnoppen an einem freien Ende eines Federelementes sitzen. Ein Drehmomentbegrenzer bzw. eine Rutschkupplung der genannten oder einer gleichwirkenden Art, beispielsweise mit linear gegeneinander beweglichen Kupplungsteilen, kann auch an anderer Stelle der Kraftübertragung zwischen Stellrad und Manschette vorgesehen sein. Die der Sollspannkraft entsprechende Manschettenweite kann beispielsweise über die Stellung des Stellrades entlang des Längsschlitzes 21 oder an anderer Stelle mit Hilfe einer Skala o. dgl. erfaßt werden und es kann damit ein (von der Höhe der Sollspannkraft geringfügig abhängiger) Dimensionswert für das Handgelenk ermittelt werden, beispielsweise der Handgelenksdurchmesser und/oder der Handgelenksumfang.

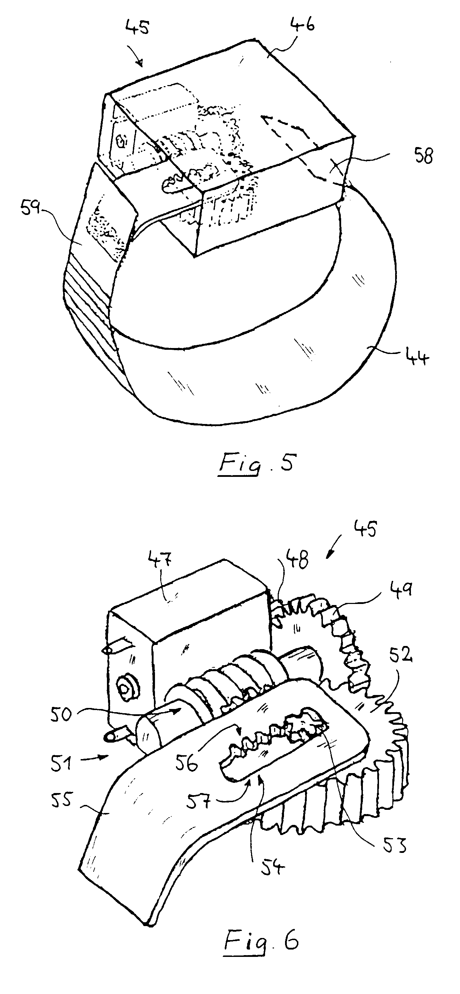

In den Figuren 5 und 6 ist eine andere Ausführungsform einer flexiblen Manschette 44 gezeigt, die mittels einer mit einem elektromotorischen Spannantrieb betriebenen Spannvorrichtung 45 mit genau vorgebbarer Spannkraft um das Handgelenk eines Patienten gelegt werden kann. In einem Gehäuse 46 der Spannvorrichtung ist ein mit Batterie oder Akkumulator gespeister Gleichstrom-Elektromotor 47 angebracht, der über einen ein Untersetzungsgetriebe bildenden ersten Zahnradsatz 48, 49 eine achsparallel mit der Elektromotorwelle drehende Schnecke 50 eines Schneckentriebs 51 drehend antreibt. Die Schnecke 50 greift in ein Schneckenzahnrad 52, das drehfest mit einem koaxialen Ritzel 53 kleineren Durchmessers verbunden ist. Das Ritzel 53 sitzt in einer nach Art einer Zahnstange geformten Längsausnehmung 54 eines gewinkelten, bandförmigen Manschettenriegels 55, greift in eine durch eine Längsseite der Ausnehmung 54 gebildete Zahnstange 56 ein und stützt sich an der gegenüberliegenden Seite an einem parallelen, geraden Längsrand 57 der Ausnehmung 54 ab, so daß das Ritzel nicht außer Eingriff mit der Zahnstange 56 geraten kann. In alternativer Ausbildung ist eine Abstützung des Manschettenriegels 55 im Gehäuse 46 vorgesehen (in den Figuren nicht dargestellt). Zusätzlich zu der zweifachen Untersetzung können eine oder mehrere Untersetzungsstufen mittels Getriebeverzahnung beispielsweise im Kraftweg vor dem Ritzel 53 vorgesehen sein.

Die Manschette 44 ist mit einem Endbereich 58 fest am Gehäuse 46 angebracht und im Bereich des anderen Endabschnitts 59 mit dem durch ein Fenster aus dem Gehäuse herausgeführten Außenabschnitt des Manschettenriegels 55 durch einen z.B. Klettverschluß werkzeuglos lösbar verbindbar. In die aus dehnungsstabilem, flexiblem Kunststoffmaterial gefertigte Manschette 44 ist eine nicht dargestellte, innenliegende flexible Druckblase integriert, die im Bereich des fest angebrachten Endabschnitts 58 unter dem Gehäuseboden bis in den Mittenbereich des Gehäuses geführt ist. Dort befindet sich ein nicht gezeigter Druckluftanschluß an die Druckblase, der mit üblichen, in dem Gehäuse 46 untergebrachten pneumatischen Elementen wie Pumpe, Druckaufnehmer und Ventil verbunden ist.

Die Befestigung der zunächst geöffneten Manschette geschieht von Hand, indem die Manschette um das Handgelenk gelegt und der Endabschnitt 59 an dem in der in Fig. 6 gezeigten Außenstellung stehenden Manschettenriegel 55 befestigt wird. Anschließend pumpt die nicht gezeigte Drucklufteinrichtung etwas Luft in die nicht dargestellte Blase bzw. Druckkammer, wobei deren Ablaßventil geschlossen ist. Nach geringfügiger Befüllung der Druckblase wird die Pumpe angehalten und automatisch die Spannvorrichtung 45 durch Einschalten des Motors 47 in Gang gesetzt. Der Motor dreht sich in die Richtung, die zu einem Einziehen des Manschettenriegels in das Gehäuse führt. Solange die Manschette noch recht locker um das Handgelenk sitzt, bleibt der Druckblasendruck relativ gering. Nähert sich bei weiterem Anziehen und Ansteigen der Spannkraft die Manschettenweite der optimalen Manschettenweite, so wird die Druckblase zusammengedrückt und ihr mittels eines Drucksensors überwachter Innendruck steigt entsprechend der steigenden Spannkraft an. Bei einem der Sollspannkraft entsprechenden Sollwert des Druckblaseninnendruckes wird der Spannvorgang durch Anhalten des Elektromotors 47 bzw. dessen Abschaltung beendet.

Die der Sollspannkraft entsprechende Manschettenweite ist bei dieser Ausführungsform ohne gesonderte Sperrglieder automatisch dadurch festgelegt, daß der Schneckentrieb als selbsthemmender Schneckentrieb ausgebildet ist. Damit hält der Mechanismus die Manschettenspannung selbsttätig ab der Abschaltung des Motors konstant. Dann wird die Druckluftpumpe wieder gestartet, so daß die Druckblase zur Durchführung des eingangs beschriebenen Meßablaufes weiter gefüllt werden kann.

Eine alternative oder zusätzliche Möglichkeit der Spannkraftbegrenzung kann durch eine den Leistungsverbrauch bzw. Motorstrom des Elektromotors 47 überwachende Sensoreinrichtung erreicht werden. Denn der zur weiteren Spannung erforderliche Motorstrom steigt mit wachsendem Lastmoment insbesondere nah der Sollspannkraft stark an, so daß aus dem Leistungsverbrauch ein Meßsignal abgeleitet werden kann, das die Abschaltung des Motors bei Erreichen der Sollspannkraft bewirkt.

Nach Abschluß der Messung kann der Gleichstrommotor 47 durch Umpolung in umgekehrter Richtung für eine begrenzte Zeit drehen, so daß der Manschettenriegel 55 motorisch wieder aus dem Gerät herausgefahren wird. Dadurch ist eine Einrichtung zum motorischen bzw. automatischen Öffnen oder Erweitern der Manschette, insbesondere nach Abschluß der Messung, geschaffen. Beim nächsten Anlegen der Manschette an den wieder voll ausgefahrenen Manschettenriegel steht dessen gesamte Zuglänge wieder zur Verfügung.

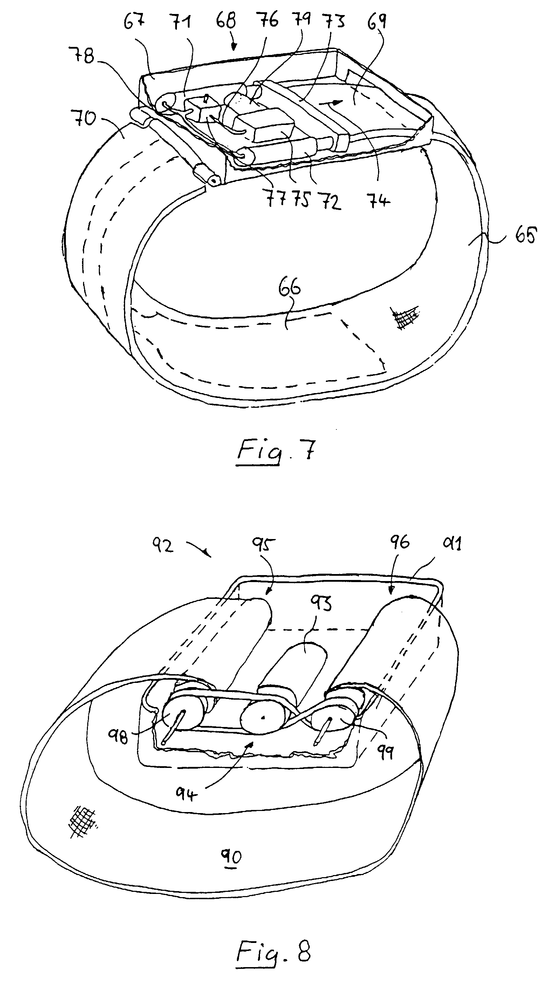

In Fig. 7 ist eine andere Ausführungsform einer flexiblen Manschette 65 mit einer integrierten flexiblen Druckblase 66 gezeigt. Die für eine Blutdruckmessung erforderliche Spannung der um ein Handgelenk zu legenden Manschette 65 wird über einen in einem flachen Gehäuse 67 untergebrachten Spannantrieb 68 mit einer pneumatischen Zugvorrichtung erreicht, die einen Endabschnitt 69 der mit dem gegenüberliegenden Endabschnitt 70 gelenkig mit dem Gehäuse verbundenen Manschette 65 in das Gehäuse einziehen kann. In dem Gehäuse sind bei der Ausführungsform zwei parallel wirkende, pneumatische Stellzylinder 71, 72 vorgesehen, die einen mit dem Manschettenendabschnitt 69 fest verbundenen Befestigungsriegel 73 in Längsrichtung 74 verschieben können. Eine über einen Elektromotor 79 betriebene Druckluftpumpe 75 ist über einen Verbindungsschlauch 76 über ein Steuerventil 77 mit den Zylindern verbunden, wobei der Schlauch 76 bei Direktanschluß auch entfallen kann. Das Steuerventil 77 leitet die Druckluft über einen bei der Ausführungsform verzweigten Schlauchanschluß 78 an die Pneumatikzylinder, die mit entsprechender Kraft die am Befestigungsriegel befestigten, parallelen Kolbenstangen in die Zylinder einzieht, wodurch die Manschettenweite verringert und deren Spannkraft bei Anliegen an ein Körperglied erhöht werden kann.

Der Druck in den Pneumatikzylindern entspricht einer Spannkraft, die auf die Manschette ausgeübt wird. Diese wird sich so lange mit geringer Kraft um das Handgelenk verengen lassen, so lange sie noch nicht fest anliegt. Bei richtigem Anliegen steigt die zur weiteren Verengung erforderliche Spannkraft bzw. Zugkraft deutlich an, was durch Überwachung des Drucks der Pneumatikseite gemessen werden kann.

Der pneumatische Druck wird vorteilhaft mit dem Drucksensor erfaßt, der ohnehin zur Detektion der Blutdruckoszillationen benötigt wird, so daß der Sensor eine Doppelfunktion erfüllen kann und eine entsprechende Einrichtung entsprechend kostengünstig herstellbar ist. Der Drucksensor ist in der Figur nicht dargestellt, kann aber beispielsweise an der Verbindungsleitung 78 zwischen Schaltventil 77 und den Pneumatikzylindern oder zwischen Pumpe 75 und Steuerventil 77 angeschlossen sein. Die Verbindungsleitungen sind nur beispielhaft schlauchartig dargestellt. Sie können auch als Kanäle in Materialblöcken ausgeführt sein, in die auch andere Pneumatikkomponenten integriert sein können.

Bei der gezeigten Ausführungsform wird der Spannprozeß bei Erreichen einer vorbestimmbaren Sollspannkraft dadurch beendet, daß der zylinderseitige Ausgang des Stellventils 77 gesperrt wird, wodurch der Zylinderinnendruck konstant gehalten wird. Entsprechend halten die Druckzylinder die Spannung der Manschette um das Handgelenk ab diesem Zeitpunkt konstant. Eine zusätzliche Sicherung zur Festlegung der Manschettenweite ist nicht erforderlich, kann aber beispielsweise in Form einer Sperrklinke oder dergleichen vorgesehen sein.

Das Steuerventil 77 wird jetzt automatisch oder manuell in eine andere Position gebracht, die einen Auslaß des Steuerventils mit der elastischen Druckblase 66 verbindet, so daß diese nun bei weiterhin laufender Pumpe 75 gefüllt werden kann. Hierzu besitzt das Ventil 77 einen zum Gehäuseboden weisenden Anschluß, der mit einem Anschlußstutzen der Manschettenblase 66 fest verbunden ist. Ab diesem Zeitpunkt wird also der Luftstrom der Pumpe in die Druckblase umgeleitet und bei Erreichen eines für die Blutdruckmessung geeigneten Maximaldruckes abgeschaltet. Der weitere Meßablauf kann wie eingangs beschrieben erfolgen.

Nach Abschluß der Messung kann das Steuerventil 77 weitergestellt werden, so daß die Druckleitung 78 bzw. die Pneumatikzylinder mit der Umgebungsatmosphäre verbunden werden. Die Pneumatikzylinder werden dadurch druckfrei und die Kolben können herausgezogen werden. Der Benutzer öffnet die Manschette manuell, indem er die notwendige Manschettenlänge aus dem Gerät herauszieht.

Bei dieser Ausführungsform wird also der Motor des Spannantriebes bei Erreichen der Sollspannkraft nicht angehalten oder abgeschaltet, sondern es wird die zunächst zur Spannung verwendete Motorkraft bzw. Druckluft durch das Steuerventil 77 auf eine andere Komponente des Meßgerätes, nämlich die expandierbare Druckblase 66 umgeleitet. Auch die Pumpe 75 erfüllt dadurch eine doppelte Funktion. Umgekehrt kann bei druckluftbetriebenen Blutdruckmeßgeräten, die ohnehin mindestens eine Pumpe und mindestens einen Drucksensor haben, allein durch geeignete Anbringung von einem oder mehreren Pneumatikzylindern in Verbindung mit einem Steuerventil eine automatische Spannung und eine automatische Spannkraftbegrenzung realisiert werden. Dies bedeutet eine Funktionalitätserweiterung unter Minimierung der Zahl hierfür erforderlicher Bauteile.

In Fig. 8 ist schematisch eine andere Ausführungsform einer verschlußfreien, flexiblen Manschette 90 eines nach dem oszillometrischen Prinzip arbeitenden Handgelenk-Blutdruckmeßgerätes gezeigt. Die in einem flachen Gehäuse 91 untergebrachte Manschettenspannvorrichtung 92 hat einen Gleichstrommotor 93, der über einen Riementrieb 94 zwei achsparallel zueinander und zur Motorwelle und gegenläufig zueinander drehende Walzen 95, 96 antreibt, auf die jeweils ein Endabschnitt der flexiblen Manschette aufwickelbar ist. Der beispielhaft und schematisch gezeigte Riementrieb 94 hat einen durch den Motor 93 angetriebenen Zahnriemen, der jeweils auf den Wellen der Walzen 95, 96 drehfest angeordnete Rollen 98, 99 gegenläufig antreibt. Bei Systemen mit mehreren Wickelwalzen kann der Riemenantrieb auch mehrere Riemeneinheiten haben. Durch die Wickelvorrichtung 92 können beide Enden der Manschette mittels der Walzen 95, 96 bei Drehung der Motorwelle gleichzeitig in das Gehäuse eingezogen werden. Bei einer anderen Ausführungsform ist ein Manschettenendabschnitt ggf. abnehmbar am Gehäuse befestigt und es wird nur ein Ende unter Aufwicklung von Manschettenmaterial eingezogen.

Über eine nicht dargestellte Meßvorrichtung kann die Kraft gemessen werden, mit der Manschettenmaterial in das Gehäuse hineingezogen wird. Mit Hilfe einer Stellvorrichtung kann das Antriebsmoment des Elektromotors 93 gesteuert werden, wobei das Antriebsmoment auf einer durch den Arm und die Manschette gebildeten Regelstrecke in eine Spannkraft gewandelt wird. Über eine Regelvorrichtung kann mit Hilfe der Meßvorrichtung und der Stellvorrichtung die maximale Kraft, mit der Manschettenmaterial in das Gehäuse eingezogen werden soll, geregelt werden, wodurch eine Einstelleinrichtung für die Sollspannkraft geschaffen ist. Der in der Steuerung realisierte Regelalgorithmus sorgt für ein Abschalten des Elektromotors bei Erreichen der gewünschten (einstellbaren) Sollspannkraft. Eine nicht gezeigte, beispielsweise an den Walzen 95, 96 angreifende, z.B. formschlüssige Arretiervorrichtung kann verhindern, daß Manschettenmaterial während der Blutdruckmessung abgewickelt wird. Das mit einem Abwickeln von Manschettenmaterial von den Walzen einhergehende Öffnen der Manschette kann motorisch unterstützt erfolgen, indem eine Umkehreinrichtung in der Steuervorrichtung für den Elektromotor dessen Drehrichtung umkehrt. Die Öffnung kann ggf. bis zum maximalen Durchmesser der Manschette erfolgen.

Die beschriebene Ausführung der als Wickelvorrichtung ausgebildeten Spannvorrichtung kann eine Vielzahl zweckmäßiger Varianten ermöglichen. So kann der Elektromotor 93 auch andere Komponenten des Blutdruckmeßgerätes, beispielsweise eine Pumpe antreiben, wobei verschiedene Komponenten des Blutdruckmeßgerätes mit beispielsweise elektromechanischen Kupplungen an den Elektromotor gekoppelt sein können. Eine wahlweise Kopplung/Entkopplung kann dazu benutzt werden, daß, beispielsweise bei Erreichen der Sollspannkraft, nur die Spannvorrichtung 92 vom Elektromotor entkoppelt werden kann und daß ggf. andere Komponenten des Meßgerätes beim Spannen der Manschette im Leerlauf betrieben werden. Diese Kopplung läßt sich robust und kostengünstig insbesondere dadurch realisieren, daß die Spannvorrichtung mit einem Freilauf versehen ist und eine oder mehrere andere Komponenten ebenfalls mit einem Freilauf versehen sind, wobei nur in einer Drehrichtung der Motorwelle gespannt wird und in der anderen Drehrichtung der Freilauf der Spannvorrichtung durchdreht und die anderen Komponenten angetrieben werden. Zum zweckmäßigen Festlegen der Manschette bei Erreichen der Sollspannkraft kann der Freilauf der Spannvorrichtung eine Sperrvorrichtung enthalten, um das Abwickeln von Manschettenmaterial vor Ende der Messung zu verhindern. Eine Verhinderung des Abwickelns vor Ende der Messung kann ggf. auch dadurch erreicht werden, daß der Elektromotor nach Spannen der Manschette nur mit einem Bruchteil des zum Spannen erforderlichen Stroms in Spannrichtung weiter betrieben wird. Auch bei dieser Ausführungsform kann, wie bereits oben beschrieben, die an der Manschette angreifende Spannkraft über den Motorstrom als Kenngröße erfaßt werden. Bei der Kraftmessung über den Motorstrom kann die Zahl von Umdrehungen der Walzen und/oder der Motorwelle erfaßt werden. Hierdurch kann der Momentenveränderung durch zunehmende Dicke der Wickelrolle Rechnung getragen werden. Es ist auch möglich, mittels einer Umdrehungszahlerfassung, die vorteilhaft auch Bruchteile von Umdrehungen berücksichtigt, die Länge des aufgewickelten Manschettenmaterials zu erfassen, wodurch eine Meßeinrichtung für den Umfang und/oder Durchmesser des Körpergliedes geschaffen werden kann. Zum Entspannen der Manschette kann vorgesehen sein, daß sich die Wickelwalzen auch ungebremst drehen können. Es ist auch möglich, eine manuell betreibbare Wickelvorrichtung zu schaffen. Zwischen Antrieb und Wickelrolle kann auch eine mechanische Drehmomentbegrenzung, z.B. nach Art der beschriebenen Rutschkupplung, vorgesehen sein.

Die beschriebenen Ausführungsformen können vorteilhaft eine stufenlose Spannkrafteinstellung ermöglichen. Eine nicht näher dargestellte, sehr einfache und kostengünstige Ausführungsform hat eine nach Art einer Handschelle mit mehreren starren, gelenkig miteinander verbundenen Teilen ausgebildete Manschette, wobei eine sägezahnartig gestalteter Manschettenendabschnitt in eine an dem anderen Endabschnitt vorgesehene Rasteinrichtung einrastet. Die automatische Spannkraftbegrenzung ist hier durch eine Kraftbegrenzung in einer der Einschubrichtung entsprechenden Längsrichtung gegeben, die ab einem bestimmten Einrastwiderstand ein weiteres Einrasten verhindert.

## Patentansprüche

1. Verfahren zum Anlegen einer Manschette eines Blutdruckmeßgerätes um ein Körperglied, insbesondere ein Handgelenk, bei dem die Manschette derart unter Aufbau einer Spannkraft um das Körperglied gelegt wird, das durch Expansion einer der Manschette zugeordneten, insbesondere in die Manschette integrierten, fluidbefüllbaren Druckblase ein blutdurchströmtes Körpergefäß drückbar bzw. abdrückbar ist,
dadurch gekennzeichnet,
daß die Spannkraft automatisch auf eine vorgebbare Sollspannkraft begrenzt wird.

2. Verfahren nach Anspruch 1,
dadurch gekennzeichnet,
daß die Spannkraft manuell erzeugt wird, insbesondere durch Bewegen eines mit der Manschette gekoppelten, vorzugsweise drehbaren Betätigungsgliedes einer Spannvorrichtung.

3. Verfahren nach dem Oberbegriff von Anspruch 1, insbesondere nach Anspruch 1 oder 2,
dadurch gekennzeichnet,
daß die Spannkraft automatisch, insbesondere elektromotorisch und/oder pneumatisch, erzeugt wird.

4. Verfahren nach einem der vorhergehenden Ansprüche,
dadurch gekennzeichnet,
daß zum Verengen der Manschette und zur Erzeugung der Spannkraft mindestens ein mit der Manschette kraftübertragend gekoppelter, manueller oder automatischer Spannantrieb betätigt wird und daß der Spannantrieb bei Erreichen der Sollkraft von der Manschette automatisch kraftentkoppelt wird.

5. Verfahren nach einem der vorhergehenden Ansprüche,
dadurch gekennzeichnet,
daß die Spannkraft mit einem automatischen Spannantrieb erzeugt wird und daß bei Erreichen der Sollspannkraft der Spannantrieb angehalten, insbesondere abgeschaltet wird.

6. Verfahren nach einem der vorhergehenden Ansprüche,
dadurch gekennzeichnet,
daß die einen automatischen Spannantrieb antreibende Kraft nach Erreichen der Sollspannkraft teilweise oder vollständig auf eine oder mehrere andere Einrichtungen des Blutdruckmeßgerätes umgeleitet wird, wobei vorzugsweise ein Fluidstrom einer fluidbetätigten Spannvorrichtung nach Erreichen der Sollspannkraft in die expandierbare Druckblase umgeleitet wird.

7. Verfahren nach einem der vorhergehenden Ansprüche,
dadurch gekennzeichnet,
daß beim Spannen der Manschette mindestens eine der auf die Manschette wirkenden Spannkraft entsprechende Kenngröße erfaßt wird und daß bei Erreichen einer der Sollspannkraft entsprechenden Sollkenngröße das Spannen der Manschette beendet wird, wobei vorzugsweise als Kenngröße ein Druck in einem Pneumatikzylinder eines pneumatischen Spannantriebes und/oder der Motorstrom eines einen Spannantrieb antreibenden Elektromotors erfaßt wird.

8. Verfahren nach einem der vorhergehenden Ansprüche,
dadurch gekennzeichnet,
daß zum Schließen der Manschette und/oder zum Erzeugen der Spannkraft mindestens ein flexibler Manschettenabschnitt, insbesondere mindestens ein Manschettenendabschnitt, insbesondere motorisch, aufgewickelt wird.

9. Verfahren nach einem der vorhergehenden Ansprüche,
dadurch gekennzeichnet,
daß eine flexible Manschette zur Verwahrung, vorzugsweise im wesentlichen vollständig, insbesondere motorisch, aufgewickelt wird.

10. Verfahren nach einem der vorhergehenden Ansprüche,
dadurch gekennzeichnet,
daß bei oder nach Erreichen der Sollspannkraft die zugeordnete Manschettenweite formschlüssig und/oder reibschlüssig festgelegt wird.

11. Verfahren nach dem Oberbegriff von Anspruch 1, insbesondere nach einem der vorhergehenden Ansprüche,
dadurch gekennzeichnet,
daß eine Erweiterung der Manschette, insbesondere nach erfolgter Messung, motorisch durchgeführt oder unterstützt wird, vorzugsweise bis zu einer maximalen Öffnungsweite der Manschette, wobei insbesondere die Motorwelle eines Motors eines Spannantriebes in umgekehrter Drehrichtung gedreht wird.

12. Verfahren nach einem der vorhergehenden Ansprüche,
dadurch gekennzeichnet,
daß bei Erreichen der Sollspannkraft ein der zugeordneten Manschettenweite zugeordneter Meßwert bestimmt wird und daß aus dem Meßwert mindestens ein Dimensionsparameter des von der Manschette umschlungenen Körpergliedes abgeleitet wird, insbesondere ein Körperglieddurchmesser und/oder ein Körpergliedumfang, wobei insbesondere der Meßwert zur Korrektur von Blutdruckmeßwerten und/oder zur Identifizierung des Benutzers verwendet wird.

13. Spannvorrichtung für eine um ein Körperglied, insbesondere um ein Handgelenk legbare Manschette eines Blutdruckmeßgerätes, wobei die Spannvorrichtung derart ausgebildet ist, daß die Manschette unter Aufbau einer Spannkraft derart um das Körperglied legbar ist, daß durch Expansion einer der Manschette zugeordneten, insbesondere in die Manschette integrierten fluidbefüllbaren Druckblase ein blutdurchströmtes Körpergefäß drückbar bzw. abdrückbar ist, gekennzeichnet durch Begrenzungsmittel zur automatischen Begrenzung der Spannkraft auf eine vorgebbare Sollspannkraft.

14. Spannvorrichtung nach Anspruch 13,
dadurch gekennzeichnet,
daß sie mindestens einen manuell oder motorisch antreibbaren Spannantrieb aufweist, der über eine Kopplung kraftübertragend mit der Manschette verbindbar ist und daß die Begrenzungsmittel mindestens eine in der Kopplung vorgesehene, vorzugsweise krafteinstellbare, Kraftübertragungsbegrenzungseinrichtung aufweist, die vorzugsweise als kraftabhängig arbeitende Entkopplungseinrichtung (40) ausgebildet ist.

15. Spannvorrichtung nach Anspruch 14,
dadurch gekennzeichnet,
daß die Kraftübertragungsbegrenzungseinrichtung nach Art einer Rutschkupplung (40) ausgebildet ist, vorzugsweise mit einem ersten Kupplungsglied (30) mit einem oder mehreren federbelasteten Vorsprüngen (34), und einem gegenüber dem ersten Kupplungsglied beweglichen, insbesondere verdrehbaren zweiten Kupplungsglied (27), das Ausnehmungen (35) zum Eingriff für die Vorsprünge aufweist, wobei insbesondere die auf die Vorsprünge wirkende Federkraft einstellbar ist.

16. Spannvorrichtung nach einem der Ansprüche 13 bis 15,
dadurch gekennzeichnet,
daß sie mindestens einen kraftübertragend mit der Manschette gekoppelten, vorzugsweise motorischen, Spannantrieb aufweist und daß die Begrenzungsmittel eine Einrichtung zum spannkraftabhängigen Anhalten, insbesondere Abschalten des Spannantriebs aufweisen.

17. Spannvorrichtung nach einem der Ansprüche 13 bis 16,
dadurch gekennzeichnet,
daß sie mindestens einen kraftübertragend mit der Manschette (65)gekoppelten, vorzugsweise motorischen und/oder pneumatischen, Spannantrieb (68) aufweist und daß vorzugsweise automatisch eingreifende, Mittel (77) zum Umlenken der den Spannantrieb antreibenden Kraft von der Manschette auf mindestens eine andere Einrichtung (66) des Blutdruckmeßgerätes bei Erreichen der Sollspannkraft vorgesehen sind.

18. Spannvorrichtung nach einem der Ansprüche 13 bis 17,
dadurch gekennzeichnet,
daß ein Spannantrieb mit mindestens einem Elektromotor (47, 79, 93), vorzugsweise ein Gleichstrommotor, vorgesehen ist, wobei vorzugsweise das Antriebsmoment des Elektromotors einstellbar ist.

19. Spannvorrichtung nach einem der Ansprüche 13 bis 18,
dadurch gekennzeichnet,
daß ein fluiddruckbetätigbarer Spannantrieb, insbesondere ein pneumatischer Spannantrieb (68), vorgesehen ist.

20. Spannvorrichtung nach einem der Ansprüche 13 bis 19,
dadurch gekennzeichnet,
daß der Spannantrieb mindestens eine Wickelvorrichtung zum Aufwickeln mindestens eines flexiblen Endabschnitts der Manschette (90) aufweist.

21. Spannvorrichtung nach einem der Ansprüche 13 bis 20,
dadurch gekennzeichnet,
daß der Spannantrieb mindestens eine elektromotorische und/oder pneumatische Zugeinrichtung (68) zum Einziehen mindestens eines Endabschnitts (69) der Manschette (65) in ein Gehäuse (67) der Spannvorrichtung aufweist.

22. Spannvorrichtung nach einem der Ansprüche 13 bis 21,
dadurch gekennzeichnet,
daß mindestens eine Feststelleinrichtung (18; 50) zur Feststellung der bei der Sollspannkraft erreichten Manschettenweite vorgesehen ist, vorzugsweise mit mindestens einer, insbesondere manuell entriegelbaren, Sperrklinke (18).

23. Spannvorrichtung nach einem der Ansprüche 13 bis 22,
dadurch gekennzeichnet,
daß eine Kraftübertragung zwischen einem Spannantrieb und der Manschette (44) mindestens einen, vorzugsweise selbsthemmend ausgebildeten, Gewindetrieb aufweist, insbesondere einen Schneckentrieb (51).

24. Spannvorrichtung nach einem der Ansprüche 13 bis 23,
dadurch gekennzeichnet,
daß mindestens eine Sensoreinrichtung zur Erfassung der Spannkraft oder mindestens einer der an der Manschette angreifenden Spannkraft zugeordneten Kenngröße vorgesehen ist, wobei vorzugsweise die Sensoreinrichtung mindestens einen Stromsensor zur Erfassung des Motorstroms eines Elektromotors des Spannantriebes und/oder mindestens einen Drucksensor zur Erfassung des Drucks in mindestens einem Pneumatikzylinder (71, 72) eines pneumatischen Spannantriebes (68) und/oder mindestens einen Drucksensor zur Erfassung des in einer mindestens teilweise fluidbefüllten Druckblase (66) beim Spannen herrschenden Druckes aufweist.

25. Spannvorrichtung nach Anspruch 24,
dadurch gekennzeichnet,
daß mindestens eine Einrichtung zur Beendigung der Manschettenverengung bei Erreichen einer der Sollspannkraft entsprechenden Sollkenngröße vorgesehen ist.

26. Spannvorrichtung nach dem Oberbegriff von Anspruch 13, insbesondere nach einem der Ansprüche 13 bis 25,
dadurch gekennzeichnet,
daß eine Einrichtung (45, 92) zum motorischen oder motorisch unterstützten Erweitern der Manschette, insbesondere nach Abschluß der Blutdruckmessung, vorgesehen ist, insbesondere mit einem Elektromotor (47, 43) mit umstellbarer Drehrichtung der Motorwelle.

27. Blutdruckmeßgerät mit einer Pumpe, einem Ventil, einem Drucksensor, einer elektronischen Steuereinheit, und einer Meßwerteanzeigeeinheit, die alle in einem Gehäuse aufgenommen sind, einer um ein Handgelenk, legbaren, vorzugsweise flexiblen Manschette, wobei die Manschette mit dem Gehäuse unmittelbar verbunden ist, bei dem die Manschette derart unter Aufbau einer Spannkraft um das Handgelenk legbar ist, das durch Expansion einer der Manschette zugeordneten, insbesondere in die Manschette integrierten, fluidbefüllbaren Druckblase ein blutdurchströmtes Körpergefäß drückbar bzw. abdrückbar ist, und mit einer an der Manschette angreifenden Spannvorrichtung zum Aufbau der Spannkraft,
dadurch gekennzeichnet,
daß die Spannvorrichtung nach einem der Ansprüche 13 bis 26 ausgebildet ist.

28. Blutdruckmeßgerät nach Anspruch 27,
dadurch gekennzeichnet,
daß es eine Anzeigeeinrichtung zur Anzeige der bei Erreichen der Sollspannkraft vorliegenden Manschettenweite und/oder mindestens eines Dimensionsparameters des von der Manschette umschlungenen Körpergliedes, insbesondere von dessen Umfang und/oder Durchmesser, aufweist.
